# FASCICULE DE BREVET EUROPEEN

(11) **EP 0 457 660 B1**
(45) Date de publication et mention de la délivrance du brevet: **12.02.1997**
(21) Numéro de dépôt: 91401228.1
(22) Date de dépôt: 13.05.1991
(51) Int. Cl.: C08F 2/32, A61L 15/00

(54) **Nouveaux polymères absorbants, leur procédé de fabrication et leur application**
Absorbierende Polymere, Verfahren zu deren Herstellung und deren Verwendung
Absorbing polymers, process for their preparation and their use

(30) Priorité: 14.05.1990 FR 9005989
(43) Date de publication de la demande: 21.11.1991
(73) Titulaire: HOECHST AKTIENGESELLSCHAFT, 65926 Frankfurt am Main (DE)
(72) Inventeur: Mallo, Paul, F-78400 Chatou (FR)

## Description

La présente invention concerne de nouveaux polymères absorbants, leur procédé de fabrication et leur application.

Des polymères réticulés, hydrophiles, insolubles dans l'eau à base d'acide acrylique et d'acrylate de métal alcalin sont largement utilisés aujourd'hui dans des articles d'hygiène en raison de leur étonnant pouvoir absorbant des fluides physiologiques : urine, sang, etc...

C'est le cas des polymères à base d'acide acrylique et d'acrylate de métal alcalin décrits par la demanderesse dans son brevet français N° 2 614 027.

Il est également connu par EP-A-243 768 de préparer des polymères absorbants, en présence de dérivés de la silice, qui ont un rôle d'agent émulsifiant. Il est également connu, par ce même document, d'incorporer au polymère déjà préparé, de fines particules de silice, afin d'améliorer ses propriétés absorbantes.

Toutefois, le marché des articles d'hygiène est demandeur de produits toujours soit plus performants, soit plus économiques, soit plus écologiques.

Or la Demanderesse a découvert avec étonnement de nouveaux polymères très absorbants présentant des propriétés absorbantes supérieures aux produits actuellement commercialisés ou décrits, tout en étant d'une part moins onéreux et, d'autre part, respectant mieux l'environnement.

Les polymères très absorbants selon la présente invention sont des polymères réticulés, en microperles, hydrophiles, insolubles dans l'eau à base d'acide acrylique partiellement salifié par un métal alcalin et de silice.

Par "microperles", l'on entend des perles sensiblement sphériques de diamètre compris entre 0,05 et 1 mm.

Par "insolubles dans l'eau", l'on entend que les polymères renferment, à la température ambiante, moins de 5 % en poids de produits solubles dans l'eau.

Dans le cadre de la présente invention, le terme "silice" désigne de la silice colloïdale, amorphe, à l'état de particules d'un diamètre moyen compris entre environ 7 et 150 nm, non agglomérées entre elles par des liaisons siloxane : Si-O-Si.

Actuellement, on ne sait pas exactement comment la silice colloïdale est fixée dans les polymères de la présente invention ; on sait cependant que les particules discrètes de silice sont uniformément réparties dans les microperles des polymères.

Parmi les polymères définis ci-dessus, on peut citer plus particulièrement les polymères acide acrylique-acrylate de métal alcalin-silice contenant pondéralement de 2 à 25 % de silice et de 98 à 75 % d'acide acrylique dont 60 à 80 % sont salifiés par un métal alcalin. Avantageusement, le métal alcalin est le sodium ou le potassium et préférentiellement, le métal alcalin est le potassium.

L'invention a plus particulièrement pour objet les polymères tels que définis ci-dessus contenant pondéralement de 5 à 20 % de silice et de 95 à 80 % d'acide acrylique dont 65 à 75 % sont salifiés par un métal alcalin.

Selon l'invention, les polymères définis ci-dessus sont susceptibles d'être préparés par un procédé de polymérisation en suspension eau dans huile réalisé en atmosphère inerte, caractérisé en ce que l'on introduit lentement, sous agitation, dans la phase huile désoxygénée de préférence parfaitement, maintenue à l'ébullition, et contenant un colloïde protecteur, une phase aqueuse obtenue extemporanément, de préférence au fur et à mesure de son introduction, à partir d'une part, d'une solution aqueuse de préférence désoxygénée contenant un ou plusieurs amorceurs de polymérisation hydrosolubles générateurs de radicaux libres et, d'autre part, d'une phase aqueuse contenant à une concentration de 50 ± 15 % en poids, la silice et les monomères choisis puis, lorsque la réaction de polymérisation est terminée, l'on élimine par distillation azéotropique les solvants jusqu'à obtention d'une suspension présentant un taux de siccité d'environ 85 ± 10 % en poids et qu'enfin l'on isole le polymère cherché, notamment par filtration.

La phase huile est constituée par un ou plusieurs hydrocarbures non miscibles à l'eau, inertes vis-à-vis des amorceurs de polymérisation et susceptibles de fournir avec l'eau un azéotrope tel que le cyclohexane ou des fractions d'essence de point d'ébullition compris entre 50 et 180°C.

Le colloïde protecteur, de préférence utilisé à la dose de 0,4 à 2 % en poids par rapport au poids des monomères est choisi parmi ceux couramment utilisés dans ce type de polymérisation en suspension (cf. Kirk-Othmer, Encyclopedia of Chemical Technology, 3ème édition, vol.1, page 400). Avantageusement, on choisira un éther de cellulose et, préférentiellement, un éthyléther de cellulose présentant un taux d'éthoxyle de 48 à 49,5 % (cf. Encyclopedia of Polymer Science and Engineering, 2ème édition, vol. 3, page 254). Le colloïde protecteur est préalablement dissous ou dispersé dans la phase huile. La réaction de polymérisation est effectuée à l'ébullition du milieu réactionnel, habituellement à la pression ambiante. Elle peut également être effectuée à une pression inférieure ou supérieure à la pression ambiante.

La réaction de polymérisation est amorcée par un ou plusieurs générateurs de radicaux libres, hydrosolubles, présentant avantageusement une demi-vie à 70°C supérieure à 2 heures. De tels agents d'amorçage sont notamment certains peroxydes minéraux comme le peroxodisulfate de sodium ou certains azoïques comme l'acide dicyano-4,4′-azopentanedioïque-4,4′. Ils sont avantageusement utilisés à la concentration de 200 à 3000 ppm par rapport au poids des monomères et avantageusement à une concentration de 500 à 1500 ppm. L'agent ou les agents d'amorçage utilisés sont dissous dans de l'eau, puis cette solution est soigneusement désoxygénée.

L'acrylate de potassium est obtenu avantageusement en solution aqueuse, par salification directe d'une solution aqueuse d'acide acrylique avec de la potasse. Cette salification est avantageusement effectuée à une température comprise entre 20°C et 35°C. Les monomères utilisés sont dissous dans de l'eau à une concentration d'environ 50 ± 15 % en poids.

La silice est apportée sous forme de suspensions aqueuses concentrées de particules non agglomérées de silice amorphe commercialisées notamment par la Demanderesse sous la désignation "KLEBOSOL".

La solution aqueuse d'amorçage et la phase aqueuse contenant les monomères et la silice sont mélangées extemporanément, en atmosphère inerte, au fur et à mesure de leur introduction dans la phase huile agitée, parfaitement désoxygénée et maintenue à l'ébullition par un chauffage extérieur, si nécessaire. Elles ne restent donc en contact que quelques secondes avant leur utilisation et elles sont introduites lentement, dans le milieu réactionnel à l'ébullition. La durée d'introduction peut varier selon les unités opératoires, mais généralement elle est comprise entre une et deux heures. En fin d'introduction, il est avantageux de maintenir le milieu réactionnel à l'ébullition sous agitation pour parfaire la polymérisation. La polymérisation terminée, on élimine les solvants réactionnels par distillation azéotropique jusqu'à obtention d'une suspension présentant un taux de siccité de 85 ± 10 % et l'on isole le produit attendu. En vue de cette isolation, la suspension est filtrée et les polymères selon l'invention ainsi recueillis sont séchés à 60°C jusqu'à un taux de siccité supérieur à 90 %. On obtient ainsi les polymères selon l'invention sous forme de microperles exemptes de fines.

Au cours de la réaction de polymérisation, les polymères formés se réticulent mutuellement spontanément pour fournir des polymères réticulés insolubles dans l'eau, à fort pouvoir hydrophile et à très faibles taux de monomères résiduels, lesquels sont toujours inférieurs à 0,01 % en poids.

La réticulation est d'autant plus favorisée que le degré de neutralisation de l'acide acrylique est bas, que la température de polymérisation est plus élevée. Les copolymères de la présente invention sont donc réticulés thermiquement et, de ce fait, deviennent insolubles dans l'eau et acquièrent des propriétés hydrophiles.

Ces propriétés sont accessibles aisément par un ensemble de tests simples.

Ainsi, la capacité d'absorption d'eau du polymère, désignée TG, est déterminée à 20 °C, en agitant durant 30 minutes, 0,4 g de polymère dans 500 g d'eau, puis en pesant le gel polymère obtenu égoutté. Le poids trouvé est ramené à 1 g de polymère sec. Les copolymères de la présente invention présentent dans ce test une capacité d'absorption de l'ordre de 300 à 700 g par gramme de polymère sec. Par "polymère sec", l'on désigne un polymère à 100 % de matières actives.

La capacité d'absorption de solution physiologique salée du polymère, désignée TGS, est déterminée à 20°C, en agitant durant 30 minutes, 2 g de polymère dans 500 g d'une solution physiologique salée puis en pesant le gel polymère obtenu égoutté. Le poids trouvé est ramené comme précédemment à 1 g de polymère sec. Les copolymères de la présente invention présentent dans ce test une capacité d'absorption de l'ordre de 40 à 70 g par gramme de polymère sec.

Le taux d'extractibles, désigné TE, est déterminé selon la méthode suivante :
- on place 1 g de polymère à tester dans 200 g de solution physiologique salée ;
- on agite cette suspension une heure à 20°C, puis on l'abandonne 15 heures au repos à 20°C ;
- on égoutte le gel polymère obtenu et on recueille le filtrat ;
- on dose sur 100 cm³ du filtrat les fonctions carboxyliques et carboxylates présentes ;
- on exprime le résultat de ce dosage en gramme de polymère dissous pour 100 g de polymère sec.

Dans ce test, les polymères de la présente invention présentent un taux d'extractibles de 1-5 %.

La capacité d'absorption par capillarité sous une charge de 15 g par cm², désignée TGC, est déterminée à 20°C selon le protocole suivant : dans un entonnoir cylindrique à plaque filtrante de diamètre 90 mm et de porosité 1, on étale successivement et uniformément, 40 g de sable de Fontainebleau de granulométrie 0,100 à 0,300 mm, 2 g de copolymère à tester et enfin 40 g de sable de Fontainebleau. On place ensuite sur la couche supérieure du sable par l'intermédiaire d'un disque de verre de diamètre 90 mm, une charge totale de 954 g, puis l'entonnoir est plongé dans un bac contenant une solution physiologique salée, à niveau constant, de manière que le niveau de l'eau affleure rigoureusement la face supérieure du fritté et on mesure la quantité de solution physiologique salée absorbée par capillarité par le copolymère en 90 minutes. On exprime le résultat en g de solution physiologique salée par gramme de polymère sec.

Les polymères de la présente invention présentent donc d'intéressantes propriétés absorbantes qui justifient leur application comme agent absorbant et l'invention a également pour objet, à titre d'agents absorbants, les polymères tels que définis précédemment, notamment pour la fabrication d'articles d'hygiène, particulièrement de couches pour bébés.

Les exemples suivants illustrent l'invention sans toutefois la limiter.

### Exemples

### Exemple 1

On disperse en atmosphère inerte :
- 3,5 g d'éthyléther de cellulose, désignée EEC, contenant de 48 à 49,5 % de groupements éthoxylés et présentant à 25°C, une viscosité de 200 mPa.s en solution à 5 % dans un mélange toluène-éthanol 80-20 en poids ;
- dans 634 g de cyclohexane, désignée CY.

Dans cette dispersion, soigneusement désoxygénée, agitée et maintenue à l'ébullition, on introduit en 90 minutes en atmosphère inerte, une solution obtenue par mélange extemporané au cours de l'introduction :
- d'une part, d'une solution désoxygénée de :
   . 0,467 g de peroxodisulfate de sodium, désigné PDS, dissous dans 10 g d'eau,
- d'autre part, 624 g d'une solution préparée extemporanément en dissolvant à une température inférieure à 30°C :
- 230 g (3,19 moles) d'acide acrylique, désigné AA, dans :
- 314 g d'une solution aqueuse de potasse contenant 128,9 g (2,3 moles) d'hydroxyde de potassium et 185 g d'eau, puis en ajoutant 80 g d'un sol de silice contenant 50 % de silice sous forme de particules d'un diamètre moyen, désigné Dm, de 50 nm, présentant un pH de 9 et stabilisé avec 0,3 % en poids d'hydroxyde de sodium (KLEBOSOL, PL 1346 Na).

La solution obtenue est ensuite soumise à une distillation azéotropique jusqu'à élimination de 240 g d'eau et, enfin, elle est refroidie à la température ambiante et filtrée. Le précipité est ensuite séché à 96 % de siccité sous vide à 60°C en étuve ventilée.

On obtient ainsi 372 g d'un polymère acide acrylique-acrylate de potassium-silice, insoluble dans l'eau, et se présentant sous forme de perles de quelques dixièmes de millimètre de diamètre. Ce polymère présente un taux de monomères résiduels inférieur à 0,005 % en poids, une capacité d'absorption d'eau d'environ 430 g par gramme, une capacité d'absorption de solution physiologique salée d'environ 53 g par gramme et une capacité d'absorption sous charge de 23,5 g par gramme.

Le taux d'extractibles, TE, est de 2,8 %.

### Exemples 2 - 10 et exemple de comparaison 11

Les exemples 2-10 et l'exemple de comparaison 11 sont réalisés en suivant le protocole opératoire décrit à l'exemple 1. Les tableaux I et II mentionnent les quantités de matières premières utilisées, exprimées en grammes ainsi que les caractéristiques des polymères obtenus. Le polymère très absorbant obtenu à l'exemple de comparaison 11 ne contient pas de silice et il est connu.

### Exemple de comparaison 12

On mélange soigneusement 11,7 g de silice présentant un diamètre moyen des particules de 10 µm avec 88,3 g du polymère très absorbant préparé à l'exemple 11. Le mélange obtenu présente un TGS de 8,3 et un TGC de 13,5, soit des valeurs très nettement inférieures à celles présentées par le polymère de départ. On constate donc que de la silice simplement mélangée avec un polymère très absorbant connu au lieu d'augmenter les caractéristiques d'absorption du polymère les diminue de façon drastique.

L'examen du tableau II permet de constater que la présence de silice combinée dans les polymères de la présente invention améliore leur capacité d'absorption sous charge, TGC, de près de 30 ± 5 %. Or, pour leur utilisation dans des articles d'hygiène, cette propriété est importante, voire essentielle. En outre, ce gain est obtenu avec moins de matières organiques, lesquelles constituent la partie la plus importante du prix de revient du polmère et le principal facteur onéreux de leur destruction après usage. Les polymères de la présente invention présentent donc des caractéristiques supérieures à ceux de l'art antérieur.

**TABLEAU I**

| N° des ex. | Phase huile | | | Phase aqueuse | | | | Sol de silice | | | Poids total |
|---|---|---|---|---|---|---|---|---|---|---|---|
| | EEC (g) | CY (g) | Poids (g) | PDS | AA | KOH | EAU | Poids | %SiO₂ | Dm | |
| 1 | 3,5 | 634 | 637,5 | 0,467 | 230 | 129,2 | 195,3 | 80 | 50 | 50 nm | 634,5 |
| 2 | 3,5 | 634 | 637,5 | 0,467 | 230 | 129,2 | 195,3 | 80 | 30 | 25 nm | 634,5 |
| 3 | 3,5 | 634 | 637,5 | 0,467 | 230 | 129,2 | 195,3 | 80 | 30 | 13 nm | 634,5 |
| 4 | 3,5 | 634 | 637,5 | 0,467 | 230 | 129,2 | 235,3 | 40 | 30 | 25 nm | 634,5 |
| 5 | 3,5 | 634 | 637,5 | 0,467 | 230 | 129,2 | 195,3 | 80 | 15 | 7 nm | 634,5 |
| 6 | 3,5 | 607 | 610,5 | 0,318 | 230 | 107,7 | 189,6 | 80 | 50 | 50 nm | 607,3 |
| 7 | 3,5 | 619 | 622,5 | 0,318 | 230 | 116,7 | 192,6 | 80 | 50 | 50 nm | 619,3 |
| 8 | 3,5 | 634 | 637,5 | 0,318 | 230 | 129,2 | 195,3 | 80 | 50 | 50 nm | 634,5 |
| 9 | 3,5 | 634 | 637,5 | 0,318 | 230 | 129,2 | 115,3 | 160 | 50 | 50 nm | 634,5 |
| 10 | 3,5 | 634 | 637,5 | 0,467 | 230 | 129,2 | 115,3 | 80 | 30 | 9 nm | 634,5 |
| 11 | 3,5 | 634 | 637,5 | 0,467 | 230 | 129,2 | 275,3 | 0 | | | 634,5 |

**TABLEAU II**

| N° | TN* | PP** | % SiO₂ | TG | TGS | TGC | TE | TMR*** |
|---|---|---|---|---|---|---|---|---|
| 1 | 72 % | 357 | 11,2 | 430 | 53 | 23,5 | 2,8 | <50 ppm |
| 2 | 72 % | 341 | 7,04 | 414 | 52 | 19,5 | | |
| 3 | 72 % | 341 | 7,04 | 426 | 52,5 | 21 | | |
| 4 | 72 % | 329 | 3,6 | 571 | 51,5 | 17,3 | 4,8 | <50 ppm |
| 5 | 72 % | 329 | 3,6 | 357 | 50 | 21,5 | 4,0 | <50 ppm |
| 6 | 60 % | 343 | 11,7 | 677 | 61 | 22,5 | | |
| 7 | 65 % | 349 | 11,4 | 608 | 58 | 22 | | |
| 8 | 72 % | 357 | 11,2 | 617 | 60 | 22 | | |
| 9 | 72 % | 397 | 20,1 | 480 | 48 | 19,5 | | |
| 10 | 72 % | 341 | 7,04 | 333 | 48 | 24 | | |
| 11 | 72 % | 317 | 0 | 447 | 57 | 17 | | |
| 12 | 60 % | 100 | 11,7 | | 8,3 | 13,5 | | |

| | | | | | | | | |
|---|---|---|---|---|---|---|---|---|
| * TN : taux de neutralisation | | | | | | | | |
| ** PP : poids de polymères secs exprimé en grammes | | | | | | | | |
| ***TMR : taux de monomères résiduels exprimé en ppm | | | | | | | | |

## Revendications

1. Polymères très absorbants, réticulés, hydrophiles, en microperle sensiblement sphériques de diamètre compris entre 0,05 et 1mm, insolubles dans l'eau à base de silice colloïdale, amorphe, à l'etat de particules d'un diamètre moyen compris entre 7 et 150nm et d'acide acrylique partiellement salifié par un métal alcalin.

2. Polymères selon la revendication 1, caractérisés en ce qu'ils contiennent pondéralement de 2 à 25 % de silice et de 98 à 75 % d'acide acrylique dont 60 à 80 % sont salifiés par un métal alcalin.

3. Polymères selon la revendication 1 ou 2, caractérisés en ce qu'ils contiennent pondéralement de 5 à 20% de silice et de 95 à 80 % d'acide acrylique dont 65 à 75 % sont salifiés par un métal alcalin.

4. Polymères selon l'une quelconque des revendications 1 à 3, caractérisés en ce que le métal alcalin est le sodium.

5. Polymères selon l'une quelconque des revendications 1 à 3, caractérisés en ce que le métal alcalin est le potassium.

6. Procédé de préparation d'un polymère selon l'une quelconque des revendications 1 à 5, par polymérisation en suspension eau dans huile réalisée en atmosphère inerte, caractérisé en ce que l'on introduit lentement sous agitation dans la phase huile désoxygénée, maintenue à l'ébullition et contenant un colloïde protecteur, une phase aqueuse obtenue extemporanément à partir d'une part d'une solution aqueuse désoxygénée contenant un ou plusieurs amorceurs de polymérisation hydrosolubles générateurs de radicaux libres et d'autre part, d'une phase aqueuse contenant, à une concentration de 50 ± 15 % en poids, les monomères choisis et la silice puis, lorsque la réaction de polymérisation est terminée, l'on élimine par distillation azéotropique les solvants jusqu'à obtention d'une suspension présentant un taux de siccité d'environ 85 ± 10 % en poids et l'on isole ensuite le polymère formé, notamment par filtration.

7. Procédé selon la revendication 6, caractérisé en ce que la phase huile est du cyclohexane.

8. Utilisation d'un polymère hydrophile selon l'une quelconque des revendications 1 à 5 comme composant absorbant.

9. Utilisation d'un polymère hydrophile selon la revendication 8 dans un article d'hygiène.

## Claims

1. Hydrophilic, crosslinked, highly absorbent polymers in the form of substantially spherical microbeads with a diameter of between 0.05 and 1 mm, which are insoluble in water, based on amorphous colloidal silica in the form of particles with a mean diameter of between 7 and 150 nm and on acrylic acid partly salified by an alkali metal.

2. Polymers according to claim 1, characterized in that they contain, by weight, from 2 to 25 % of silica and from 98 to 75 % of acrylic acid of which 60 to 80 % is salified by an alkali metal.

3. Polymers according to claim 1 or 2, characterized in that they contain, by weight, from 5 to 20 % of silica and from 95 to 80 % of acrylic acid of which 65 to 75 % is salified by an alkali metal.

4. Polymers according to any one of claims 1 to 3, characterized in that the alkali metal is sodium.

5. Polymers according to any one of claims 1 to 3, characterized in that the alkali metal is potassium.

6. Process for the preparation of a polymer according to any one of claims 1 to 5, by water-in-oil suspension polymerization carried out in inert atmosphere, characterized in that an aqueous phase obtained extemporaneously from, on the one hand, a deoxygenated aqueous solution containing one or a number of water-soluble polymerization initiators generating free radicals and, on the other hand, from an aqueous phase containing, in a concentration of 50 ± 15 % by weight, the chosen monomers and the silica is introduced slowly with stirring into the deoxygenated oil phase, kept boiling and containing a protective colloid, and then, when the polymerization reaction is finished, the solvents are removed by azeotropic distillation until a suspension is obtained which has a dryness value of approximately 85 ± 10 % by weight and the polymer formed is subsequently isolated, especially by filtration.

7. Process according to claim 6, characterized in that the oil phase is cyclohexane.

8. Use of a hydrophilic polymer according to any one of claims 1 to 5 as an absorbent component.

9. Use of a hydrophilic polymer according to claim 8 in an article of hygiene.

## Patentansprüche

1. Hochabsorbierende, vernetzte, hydrophile in Form von im wesentlichen kugelförmigen Mikroperlen mit einem Durchmesser zwischen 0,05 und 1 mm vorliegende, wasserunlösliche Polymere auf der Basis von amorpher, kolloidaler Kieselsäure in Form von Teilchen mit einem mittleren Durchmesser zwischen 7 und 150 nm und mittels eines Alkalimetalls teilweise in die Salzform überführter Acrylsäure.

2. Polymere nach Anspruch 1, dadurch gekennzeichnet, daß sie, bezogen auf das Gewicht, 2 bis 25% Kieselsäure und 98 bis 75% Acrylsäure enthalten, die zu 60 bis 80% mittels eines Alkalimetalls in die Salzform überführt worden ist.

3. Polymere nach Anspruch 1 oder 2, dadurch gekennzeichnet, daß sie, bezogen auf das Gewicht, 5 bis 20% Kieselsäure und 95 bis 80% Acrylsäure enthalten, die zu 65 bis 75% mittels eines Alkalimetalls in die Salzform überführt worden ist.

4. Polymere nach einem der Ansprüche 1 bis 3, dadurch gekennzeichnet, daß es sich bei dem Alkalimetall um Natrium handelt.

5. Polymere nach einem der Ansprüche 1 bis 3, dadurch gekennzeichnet, daß es sich bei dem Alkalimetall um Kalium handelt.

6. Verfahren zur Herstellung eines Polymers nach einem der Ansprüche 1 bis 5 durch Wasser-in-Öl-Suspensionspolymerisation, die unter einer Schutzgasatmosphäre erfolgt, dadurch gekennzeichnet, daß man in die von Sauerstoff befreite Ölphase, die am Sieden gehalten wird und ein Schutzkolloid enthält, unter Rühren langsam eine wäßrige Phase einträgt, die einerseits aus einer von Sauerstoff befreiten wäßrigen Lösung, die einen oder mehrere wasserlösliche, freie Radikale bildende Polymerisationsinitiatoren enthält, und andererseits aus einer wäßrigen Phase, die die gewählten Monomere und die Kieselsäure in einer Konzentration von 50 ± 15 Gew.-% enthält, in-situ hergestellt wurde, und anschließend nach Beendigung der Polymerisationsreaktion die Lösungsmittel azeotrop abdestilliert, bis man eine Suspension mit einem Trockengehalt von etwa 85 ± 10 Gew-% erhält, und das gebildete Polymer sodann, insbesondere durch Filtration, isoliert.

7. Verfahren nach Anspruch 6, dadurch gekennzeichnet, daß die Ölphase aus Cyclohexan besteht.

8. Verwendung eines hydrophilen Polymers nach einem der Ansprüche 1 bis 5 als absorbierende Komponente.

9. Verwendung eines hydrophilen Polymers nach Anspruch 8 in Hygieneartikeln.
